# EUROPÄISCHE PATENTSCHRIFT

(11) **EP 3 136 963 B1**
(45) Veröffentlichungstag und Bekanntmachung des Hinweises auf die Patenterteilung: **04.12.2019**
(21) Anmeldenummer: 15721570.8
(22) Anmeldetag: 28.04.2015
(51) Int. Cl.: A61B 5/11, A61B 3/113, G02C 7/04, G02C 7/08

(54) **VERWENDUNG VON MITTELN ZUR STEUERUNG DER AKKOMMODATIONSBEDARFSERFASSUNG IN EINEM KÜNSTLICHEN AKKOMMODATIONSSYSTEM**
USE OF MEANS FOR CONTROLLING THE ACCOMMODATION DEMAND DETECTION IN AN ARTIFICIAL ACCOMMODATION SYSTEM
UTILISATION DE MOYENS POUR COMMANDER LA DÉTECTION DES BESOINS D'ACCOMMODATION DANS UN SYSTÈME D'ACCOMMODATION ARTIFICIEL

(30) Priorität: 30.04.2014 DE 102014106036
(43) Veröffentlichungstag der Anmeldung: 08.03.2017
(73) Patentinhaber: Karlsruher Institut für Technologie (KIT), 76131 Karlsruhe (DE)
(72) Erfinder: GUTH, Helmut, 76344 Eggenstein-Leopoldshafen (DE); GENGENBACH, Ulrich, 75196 Remchingen (DE); BRETTHAUER, Georg, 76139 Karlsruhe (DE); ROTHMUND, Jan, 74523 Schwäbisch Hall (DE); KOKER, Liane, 76297 Stutensee (DE); NAGEL, Jörg, 76344 Eggenstein-Leopoldshafen (DE); MARTIN, Thomas, 76139 Karlsruhe (DE); BECK, Christoph, D-73732 Esslingen-Liebersbronn (DE)
(86) Internationale Anmeldenummer: PCT/EP2015/000868
(87) Internationale Veröffentlichungsnummer: WO 2015/165584

(56) Entgegenhaltungen:
- DE-B3-102007 008 375
- US-A1- 2003 169 907
- US-A1- 2012 140 167
- FLIEDNER JAN ET AL: "The pupil can control an artificial lens intuitively", INVESTIGATIVE OPTHALMOLOGY & VISUAL SCIENCE, ASSOCIATION FOR RESEARCH IN VISION AND OPHTHALMOLOGY, US, vol. 55, no. 2, 4 February 2014 (2014-02-04), pages 759-766, XP009507588, ISSN: 1552-5783, DOI: 10.1167/IOVS.13-12162
- KRUG M ET AL: "Enhanced power management unit for the Artificial Accommodation System", BIOMEDIZINISCHE TECHNIK. BIOMEDICAL ENGINEERING JUN 2013,, vol. 58, no. Suppl. 1, 7 September 2013 (2013-09-07), page 2pp, XP009507587, ISSN: 1862-278X, DOI: 10.1515/BMT-2013-4371

## Beschreibung

Die Erfindung betrifft Mittel zur Steuerung, vorzugsweise Triggerung der Akkommodationsbedarfserfassung in einem ophthalmischen technischen System, insbesondere einem künstlichen Akkommodationssystem gemäß dem ersten Patentanspruch. Es dient der bedarfsorientierten Beeinflussung des ophthalmischen technischen Systems durch bewusste oder unbewusste körpereigene Signale oder Umwelteinflüsse, die selbst nicht direkt für die Erfassung eines Akkommodationsbedarfs nutzbar sein müssen. Weiterhin betrifft die Erfindung eine Kontaktlinse gemäß des zehnten Patentanspruchs.

Das menschliche Auge ist ein natürliches optisches System, das Objekte mit Hilfe mehrerer lichtbrechender Grenzflächen scharf auf der Netzhaut (Retina) abbildet. Ändert sich die Gegenstandsweite des betrachteten Objektes, ist es für eine Abbildung mit gleich bleibender Schärfe auf der Netzhaut notwendig, dass sich das Abbildungsverhalten des optischen Systems ändert. Beim menschlichen Auge wird dies durch eine Verformung der Linse mit Hilfe des Ziliarmuskels (Musculus Ciliaris) realisiert, wodurch sich im Wesentlichen die Form und die Lage der Linsenvorder- und -rückseite ändern (Akkommodation).

Ein künstliches Akkommodationssystem ist ein ophthalmisches technisches System (OTS), d.h. ein künstliches optisches System, das ein Linsensystem mit mindestens einer optischen Linse mit verstellbarer Brennweite aufweist. Es zeichnet sich dadurch aus, dass es entweder im Auge eingebracht ist (z.B. als Implantat) oder in engem Kontakt mit okularem Gewebe bzw. okularen Fluiden steht (auf dem Auge bzw. zwischen den Augen und anderem Körpergewebe angebracht). Es umfasst - wie beispielhaft in der DE 10 2005 038 542 A1 dargestellt - neben einem verstellbaren Linsensystem ein Informationserfassungssystem, ein Informationsverarbeitungssystem, ein Energieversorgungssystem und ein Befestigungssystem. Das Informationserfassungssystem dient dabei der Erfassung von Messsignalen, aus denen das Informationsverarbeitungssystem einen Akkommodationsbedarf ermittelt und als Stellsignale zu Stellaktoren im Linsensystem weitergeleitet werden. Der Akkommodatiönsbedarf ist eine Größe, die in einem natürlichen Regelkreis als Stellgröße für das Linsensystem zur Einstellung einer bestimmten Brennweite zur Scharfstellung eines anvisierten Objekts durch ein Auge erforderlich ist. Im natürlichen Auge ist die Bestimmung des Akkommodationsbedarfs fester Bestandteil der Augenmotorik, wobei der Ziliarmuskel als Aktor für die Brechkraftanpassung der Linse dient, aber auch als Informationsquelle für die vorgenannten Messsignale heranziehbar ist.

Wie beispielhaft in DE 10 2005 038 542 A1 dargestellt, kann durch die Implantierung eines künstlichen Akkommodationssystems als autonom arbeitendes Implantat in das menschliche Auge die altersbedingt (Presbyopie = Alterssichtigkeit) oder bei einer Kataraktoperation (Grauer Star Operation) verloren gegangene Akkommodationsfähigkeit wiederhergestellt werden. Es ist vorgesehen, es vollständig im Kapselsack des menschlichen Auges (anstelle der natürlichen Augenlinse) einzusetzen.

Ferner sind z.B. aus US 6.851.805 B2 sowie US 2009/0015785 A1 intelligente Kontaktlinsen bekannt, bei der das Akkomodationssystem in eine Vorsatzlinse für das Auge wie z.B. eine Brille oder eine Kontaktlinse direkt auf dem Augapfel integriert sind.

Die US 6.851.805 B2 beschreibt eine akkommodierende Kontaktlinse mit integrierten Sensoren und Energieversorgung. Eine Triggerung der Akkommodationsbedarfserfassung ist nicht vorgesehen, sondern vielmehr eine direkte Zuordnung von Einflüssen und Augenstellungen auf den konkreten Akkommodationsbedarf. Für eine Erkennung der Blickrichtung und der Aktivität des Nutzers sind z.B. ein Neigungssensor (Neigungsschalter) und ein Gyroskop vorgesehen. Die Sensorsignale werden zu Aktoren für die Verstellung der Brennweite weitergeleitet. Das Konzept schließt eine Kommunikation zwischen zwei Kontaktlinsen wie auch eine Erfassung komplexer Bewegungsmuster des Nutzers aus. Der Akkommodationsbedarf wird ausschließlich und direkt nur anhand der Messwerte der Sensoren der zugehörigen Linse bestimmt.

Die US 2009/0015785 A1 offenbart beispielhaft ein Linsensystem (Intraokularlinse, Kontaktlinse, Hornhaut-Inlay oder Brille) mit in der Brechkraft einstellbaren konzentrischen Ringlinsen, die entsprechend der Umgebungsleuchtdichte oder Pupillenweite ansteuer- und einstellbar sind. Zur Erfassung der Umgebungsleuchtdichte werden insbesondere in der Linse integrierte Photodetektoren vorgeschlagen. Auch in diesem System wird eine Akkommodationsbedarfserfassung ausschließlich nur mit den Sensoren des zugehörigen Linsensystems bestimmt.

Die vorgenannten Systeme nutzen Sensoren, die direkt oder indirekt Steuersignale für ein verstellbares Linsensystem erzeugen. Die Signale repräsentieren damit den Akkommodationsbedarf, d.h. die einzelnen Signalwerte sind über mathematische Beziehungen jeweils mit einer einzustellenden Brennweite und / oder einer anderen optischen Einstellung im Linsensystem gekoppelt.

Ferner beschreibt die US 2012/0140167 A1 eine Kontaktlinse und ein intraokulares Linsensystem mit zwei über verschiedene alternative Aktorkonzepte einstellbaren Brennweiten. Die Umschaltung erfolgt vorzugsweise aufgrund erfasster Augenlidbewegungen, vorzugsweise mit systemintegrierten Photosensoren, die die Lidabschattung erkennen. Alternativ hierzu werden auch Augenbewegungen mit Beschleunigungssensoren oder Entfernungsmesser für eine Umschaltung vorgesehen. Für die Bestimmung des Akkommodationsbedarfs wird ferner optional der Einsatz eines Entfernungsmessers vorgeschlagen. Eine Verwendung von Mitteln für eine Erzeugung von Steuersignalen zur Einstellung oder Anregung von Betriebszuständen in einem ophthalmischen technischen System gemäss der Präambel von Anspruch 1 ist aus der folgenden Veröffentlichung bekannt: KRUG M ET AL: "Enhanced power management unit for the Artificial Accommodation System", BIOMEDIZINISCHE TECHNIK, JUN 2013, Bd. 58, Suppl. 1.

Von einem Akkommodationsbedarf ist die Akkommodationsbedarfserfassung zu unterscheiden. Eine Akkommodationsbedarfser-fassung umfasst die Initiierung einer Messdatengenerierung sowie die Auswertung der Signale mit dem Ziel der Ableitung eines Akkommodationsbedarfs Sie steuert die Erfassung oder Generierung der Signale, vorzugweise Messsignale, die für den erforderlichen Akkommodationsbedarf zur Einstellung des Linsensystems benötigt werden.

Eine Beeinflussung der Akkommodationsbedarfserfassung in einem künstlichen Akkommodationssystem durch äußere Einflüsse oder durch das Nutzerverhalten ist nicht bekannt. Die Systeme differenzieren beispielsweise nicht, ob eine Erfassung des vorgenannten Akkommodationsbedarfs an sich überhaupt notwendig erscheint. Üblicherweise erfolgt die Erfassung laufend beispielsweise in vorgegebenen Zeitabständen. Dies bedeutet jedoch nicht nur einen erhöhten Energieverbrauch, sondern auch mit einer laufenden Nachjustierung eine gewisse vom Nutzer ggf. wahrnehmbare Unruhe.

Davon ausgehend liegt die **Aufgabe der Erfindung** darin, Mittel zur Steuerung der Akkommodationsbedarfserfassung in einem künstlichen Akkommodationssystem oder auch anderen ophthalmischen technischen Systemen (OTS) vorzuschlagen, durch die eine signifikante Energieeinsparung des' Akkommodationssystems (oder OTS) ohne eine nennenswerte Einschränkung des Nutzers, bzw. eine Steigerung des Nutzerkomforts ermöglicht wird.

Die Aufgabe wird mit den Mitteln und der Kontaktlinse gemäß den Merkmalen in Anspruch 1 gelöst. Unteransprüche geben vorteilhafte Ausgestaltungen wieder.

Zur Lösung der Aufgabe werden Mittel zur Steuerung der Akkommodationsbedarfserfassung in einem künstlichen Akkommodationssystem (oder OTS) vorgeschlagen. Diese Mittel greifen somit in den Regelkreis eines Akkommodationssystems ein, indem sie körpereigene Signale und/oder Umweltsignale erfassen und die damit erzeugten Messsignale nutzen, um eine Erfassung des Akkommodationsbedarfs auszulösen, zu unterbrechen oder auf eine andere Weise zu beeinflussen. Die Messsignale werden zu Steuersignalen, vorzugsweise zu Triggersignalen für die Erfassung des Akkommodationsbedarfs für das Akkommodationssystem oder OTS, d.h. nicht zu Steuersignalen zur Einstellung eines Akkommodationsbedarfs eines Linsensystems gewandelt. Bei dieser Wandlung erfolgt eine Erkennung und Zuordnung der Messsignale zu vorgegebenen Verhaltensmustern des Akkommodationssystemträgers bzw. OTS-Trägers. Entsprechend dieser Zuordnung erfolgt eine Generierung eines ebenfalls vorgegebenen Steuersignals für die Erfassung des Akkommodationsbedarfs.

Die Steuersignale sind vorzugsweise Triggersignale für die Aktivierung oder Deaktivierung einer Akkommodationsbedarfserfassung oder Akkommodationsbedarfserfassungsfolge im genannten Regelkreis des Akkommodationssystems (oder OTS), einer beschleunigten oder verzögerten Taktfolge für die Akkommodationsbedarfserfassung oder für eine Unterbrechung der Akkommodationsbedarfserfassung. Dies bewirkt eine bedarfsorientierte Steuerung des Akkommodationssystems (oder OTS) mit dem Ziel der Energieeinsparung, einer längeren Lebensdauer oder eines verbesserten Nutzerkomforts.

Die Steuersignale dienen somit der Einstellung oder Anregung von Betriebszuständen, insbesondere:
1) Triggerung einer Akkommodationsbedarfserfassung,
2) Eigenschaften der Akkommodationsbedarfserfassung (z.B. Frequenz oder Frequenzentwicklung der Bedarfsmessung),
3) Beliebige Steuerung einer Akkommodationsbedarfserfassung (z.B. Ein-/Aus-/Umschälten).

Die Steuersignale umfassen anders als bei bekannten Systemen keine Signale für eine direkte Einstellung einer bestimmten Brennweite im Akkommodationssystem. Sie dienen dem Einstellen und Schalten von Betriebszuständen sowie dem Ändern von ablaufrelevanten Betriebsparametern des Akkommodationssystems. Ein Betriebszustand umfasst beispielsweise eine Einfrieren oder ein Anfahren einer eingestellten bzw. vorgegebenen Brennweite solange, bis die Steuerung eine Aufhebung dieses Betriebszustands, oder einen anderen Betriebszustand antriggert. Eine Steuerung wird z.B. dann benötigt, wenn ein System ein- oder ausgeschaltet bzw. zwischen verschiedenen Betriebsarten gewechselt werden soll, z.B. der nutzerinitiierte Wechsel von Fern- auf Nahsicht (vergleichbar mit dem Neigen des Kopfs beim Tragen einer Gleitsichtbrille) und umgekehrt.

Die Mittel umfassen zur Lösung der vorgenannten Aufgabe mindestens einen Sensor zur Erfassung einer körpereigenen Signalfolge oder einer Umfeldsignalfolge und Wandlung dieser in Messsignale sowie mindestens eine Erfassungseinheit zur Wandlung der Messsignale in ein Steuersignal, das die Akkommodationsbedarfserfassung beeinflusst.

Der Sensor dient der Signalaufnahme, d.h. ein System, das die physikalische Erfassung von Augen- bzw. Lidschlagbewegungen oder Umfeldsignalen ermöglicht. Augenbewegungen lassen sich mit Hilfe der Größen Position, Geschwindigkeit und Beschleunigung der Augen in Bezug auf ein kopffestes Referenzsystem charakterisieren. Geeignete Sensoren zur Erfassung einer oder mehrerer dieser Größen, sind z.B. Beschleunigungssensoren, Drehratensensoren, Magnetfeldsensören oder optische Sensoren. Lidschlagbewegungen lassen sich unter anderem durch den Öffnungsgrad der Augen quantitativ charakterisieren, wofür vorzugsweise kapazitive, taktile oder optische Sensorkonzepte herangezogen werden.

Die Erkennung eines Lidschlags erfolgt vorzugsweise durch einen Schaltvorgang, der nur zwischen einem offenen und einem geschlossenen Lid differenziert und z.B. den Müdigkeitsgrad des Trägers des Linsensystems oder die Umgebungshelligkeit unberücksichtigt lässt. Für eine Erfassung genügt hierbei oftmals nur ein Sensor.

Die Erfassung der Umgebungshelligkeit ist beispielsweise für die Wichtung eines Schärfentiefebereichs und damit indirekt auch einer Akkommodationsbedarfserfassung erforderlich, was durch die Erkennung des Lidschlags allein nicht möglich ist.

Der Öffnungsgrad des Lidschlags dagegen hängt von diesen vorgenannten unberücksichtigten Einflüssen ab und wird grundsätzlich auch durch vorgenannte Sensorkonzepte erfasst. Jedoch ist eine differenziertere Erfassung der Lidpositionen erforderlich, was pro Lid mit zunehmender Erfassungsgenauigkeit auch eine zunehmende Anzahl von Einzelsensoren, angeordnet als Array, als Zeile und/oder an unterschiedlichen Positionen am Lid erfordert.

Die Erfassungseinheit weist eine Signalerkennung auf, die die Messsignale mit Referenzsignalbandbreiten, die die vorgenannten Verhaltensmuster des Trägers des Akkommodationssystems oder OTS wiedergeben, vergleicht und bei einer Überdeckung der Messsignale durch eine vorgegebene Referenzsignalbandbreite dieses Verhaltensmuster erkennt und ein Steuersignal erzeugt.

Im Gegensatz zu einem einfachen Schwellwert weist eine Referenzsignalbandbreite nicht nur einen Wert auf, sondern gibt eine Bandbreite im Sinne eines Toleranzbereichs um einen im Idealfall zu erwartenden Wert körpereigener Signale und/oder Umweltsignale, d.h. um ein Referenzsignal an. Für jedes mögliche Steuersignal wird eine zugehörige Referenzsignalbandbreite definiert, die das idealisierte Signal (Referenzsignal) und den entsprechend zugehörigen Toleranzbereich umfasst. Die erhaltenen Messsignale werden mit den durch die Referenzsignalbandbreite umschlossenen Werten verglichen. Liegt ein Messsignal vollständig innerhalb des durch die Referenzsignalbandbreite aufgespannten Wertebereichs, gilt das entsprechende Referenzsignal als erkannt und das zum Referenzsignal gehörende Steuersignal wird durch die Erfassungseinheit erzeugt. Die zu erwartenden körpereigenen Signale und/oder Umweltsignale sind dabei vorzugsweise auch sich zeitlich entwickelnde Signalfolgen (Referenzsignalfolge) vorzugsweise im Sinne von Signalmustern. Die Referenzsignalbandbreite ist nicht zu verwechseln mit einer Bandbreite im Frequenzbereich, sondern entspricht einer vorzugsweise exakten Referenzsignalfolge erweitert um einen sich vorzugsweise ebenfalls zeitlich entwickelnden Toleranzbereich um diese Referenzsignalfolge. Die Referenzsignalfolge bildet zusammen mit dem zeitlich sich verändernden Toleranzbereich eine chronologisch sich entwickelnde Referenzsignalbandbreite. Der Toleranzbereich ermöglicht die Erkennung valider Messsignale und Messsignalfolgen in der Gegenwart von Störungen, Rauschen und einer Variabilität bedingt durch nicht ideale Umgebungsbedingungen bzw. eine nicht ideale Ausführung von Mustern. Somit müssen Referenzsignal oder Referenzsignalfolge und Messsignal oder Messsignalfolge nicht vollständig, sondern lediglich zu einem festzulegenden Mindestgrad deckungsgleich sein. Die Referenzsignalbandbreiten stellen demnach die Menge aller um ihren jeweiligen Toleranzbereich erweiterten Referenzsignale und Referenzsignalfolgen dar.

Eine bevorzugte Ausführung der Signalerkennung umfasst eine Mustererkennung mit Auslöser für die Auslösung eines Steuersignals sowie eine optional vorgeschaltete Segmentierung der Messsignale.

Die Segmentierung stellt einen optionalen ersten Signalverarbeitungsschritt dar, durch welchen ein Messsignal wie z.B. ein durch eine Augenbewegung hervorgerufenes Beschleunigungssignal in einzelne charakteristische Segmente, z.B. ein Beschleunigungs- und ein Verzögerungssegement unterteilt werden. Die Segmentierung ist z.B. beim Einsatz trainingsdatenbasierter Entscheidungsnetzwerke (z.B. künstliche neuronale Netze) ersetzbar.

Die Mustererkennung ermöglicht eine Interpretation der Messsignale und/oder ggf. der Segmente bezüglich vordefinierter Bewegungsmuster. Die Messsignale werden dabei mit einer vorzugsweise chronologisch sich entwickelnden Referenzsignalbandbreite verglichen und bei einer Überdeckung der Messsignale durch eine vorgegebene Referenzsignalbandbreite das Steuersignal erzeugt. Somit erfolgt in diesem Signalverarbeitungsschritt eine Differenzierung zwischen natürlichen Bewegungen und solchen Bewegungen, die der Nutzer bewusst zum Zwecke der Steuerung ausübt. Der Auslöser dient der Zuordnung eines Bewegungsmusters zu einer definierten Steueraktion. Er erfüllt somit die Aufgabe einer Ereignistriggerung. Die Steueraktion erfüllt die Umsetzung des Steuerbefehls. Das Resultat der Steueraktion ist das Steuersignal z.B. die Einstellung eines Betriebszustands oder die Änderung von Betriebsparametern.

Die Erfindung ist auf dem Gebiet der Mensch-Maschinen-Interaktion angesiedelt. Als Mittel zur Interaktion zwischen Mensch und Maschine sieht die Erfindung dabei die Nutzung von körpereigenen Signalen und/oder Umweltsignalen vor, die im Auge oder in unmittelbarer Angrenzung an das Auge detektiert werden.

Die Mittel erfassen vorzugsweise bewusste oder unbewusste Augenbewegungen oder alltägliche Verhaltensmuster des Trägers des Akkommodationssystems oder OTS, die in der Signalerkennung der Erfassungseinheit interpretiert und eingeordnet werden und auf der Basis dieser Einordnung das Steuersignal generiert wird. Ein Steuersignal ist entweder ein einmaliges Signal oder eine Signalfolge.

Unbewusste Augenbewegungen sind vorzugsweise Sakkaden- und reflexartige Lidschlagbewegungen. Bewusste Augenbewegungen sind vorzugsweise bewusste Bewegungen und Bewegungsfolgen der Augen und/oder der Lider. Sie umfassen insbesondere auch ein vollständiges Schließen der Augen oder auch unnatürliche Augenbewegungen wie z.B. das Blinzeln in zeitlichen Codes (z.B. Morsecodes). Sie lösen vorzugweise Triggersignale für die Akkommodationsbedarfserfassung als Steuersignal aus, wobei der Begriff des Triggersignals im Rahmen der Anmeldung sowohl Einzeltriggersignale als auch Triggersignalfolgen umschließt.

Alltägliche Verhaltensmuster charakterisieren typische Tätigkeiten oder Haltungen des Trägers des Akkommodationssystems oder OTS, die nach wiederkehrendenMustern ablaufen und über diese auch erkennbar sind. Typische Verhaltensmuster sind z.B. Lesen, Computerarbeit, Fernsehen, körperliche Sportarten wie Laufen, Autofahren, Hausarbeit, Warten, Beobachten und anderen Tätigkeiten, bei denen sich die angestrebte Brennweite des Auges unifokal nicht oder nur in einem geringen Maße ändert, bifokal zwischen zwei Brennweiten hin- und hergeschaltet wird oder eine bestimmte Fokuseinstellung nicht erforderlich ist (z.B. beim Schlafen). Ggf. erforderliche geringe Änderungen der gewünschten Brennweite befinden sich vorzugsweise im Schärfentiefebereich und werden durch diesen abgedeckt. Folglich werden diese Brennweiten im Akkommodationssystem vorzugsweise als festeEinstellungen implementiert, d.h. der Akkommodationsbedarf muss nicht bei jeder Wiederholung einer dieser Einstellung neu ermittelt werden. Folglich umfasst das Steuersignal ein Stellsignal (Auslösesignal) für einen vorgegebenen Brennweitenbereich oder eine vorgegebene, vorzugsweise eine reduzierte Frequenz in der Akkommodationsbedarfserfassung im Akkommodationssystem. Ein Hin- und Herschalten zwischen zwei Brennweiten ist z.B. durch ein zusätzliches Steuersignal, das durch eine Lidschlag oder charakteristische Augenbewegung auch ohne eine Akkommodationsbedarfserfassung auslösbar.

Umfeldeinflüsse sind äußere Einflüsse, beispielsweise Dunkelheit, die durch eine Umfeldsignalfolge wiedergegeben werden. Das Steuersignal umfasst vorzugsweise ein Stellsignal (Auslösesignal) für einen vorgegebenen Brennweitenbereich oder eine vorgegebene, vorzugsweise eine reduzierte Frequenz in der Ackommodatiönsbedarfserfassung im Akkommodationssystem.

Im Einzelnen sind folgende Verhaltensmuster sowie Mittel zur Erfassung dieser und Steuerung der Akkommodationsbedarfserfassung in einem künstlichen Akkommodationssystem (oder OTS) vorteilhaft:

### 1. Unbewusste Augenbewegungen:

Zu den unbewussten Augenbewegungen zählen unbewusste Sakkaden wie Drehungen des Augapfels oder Lidschlussbewegungen. Sakkaden sind Augenbewegungen, bei denen sich vorzugsweise der Augapfel drehend bewegt. Änderungen des Akkommodationsbedarfs gehen in der Regel mit Sakkadenbewegungen des Auges einher. Sackaden treten mit einer durchschnittlichen Frequenz unterhalb 10 Hz, vorzugsweise von 0,2 bis 4 Hz auf (Sakkadendauer typischerweise ca. 50-250 ms). Unabhängig von der Amplitude weisen Sakkaden im Beispiel einer Augendrehung eine anfängliche Drehbeschleunigung von ca. 20000 deg/s² auf. Auf einer Kreisbahn um den Augendrehpunkt mit einem angenommenen Radius von ca. 10 mm (Entfernung Implantat von Drehpunkt) resultiert dies in einer tangentialen Beschleunigung von ca. l g (einfache Schwerkraftbeschleunigung).

Die Bewegungen des Augapfels sind als körpereigene Signalfolgen vorzugsweise mit Beschleunigungssensoren vorzugsweise auf der Augapfeloberfläche erfassbar. Diese nehmen die Beschleunigungsbewegung entweder direkt auf oder alternativ indirekt über Gyroskope oder Magnetfeldsensoren relativ zu einem ortsfesten System z.B. Magnetfeldsystem.

Beschleunigungssensoren sind vorzugsweise Massenträgheitssensoren herkömmlicher Art, für einen Einsatz im Rahmen der Erfindung vorzugsweise piezoelektrische, kapazitive oder induktive Sensoren, weiter bevorzugt miniaturisierbare fotolithographisch oder in Siliziumtechnik herstellbare Sensoren.

Zu langsame Beschleunigungen wie langsame Folgebewegungen, können jedoch zu Fehlmessungen führen und lösen z.B. auch keine sakkadenbasierte Triggerung aus. In diesem Fall wird vorgeschlagen, dass in Abwesenheit von geeigneten durch Sakkaden hervorgerufenen Beschleunigungssignalen oder -folgen oder Steuersignalen wie Triggersignalen sich im Akkommodationssystem eine vorgegebene niedrige Frequenz von 0,25 bis 2,0 Hz, bevorzugt zwischen 0,5 und 1,5 Hz bei der Akkommodationserfassung automatisch einstellt, da zwischen zwei Sakkaden nur mit geringen Unterschieden des Akkommodationsbedarfs ausgegangen werden kann.

Ferner zählen spontane Lidschlussbewegungen (z.B. Blinzeln) zu den unbewussten Augenbewegungen. Unbewusste Lidschlagbewegungen gehen in der Regel einher mit Sakkaden (Blickwechselbewegungen), da während Sakkaden ohnehin die visuelle Wahrnehmung unterdrückt ist. Lidschlussbewegungen sind vorzugsweise durch lichtempfindliche Sensoren erfassbar, die bevorzugt am Auge wie z.B. auf einer Kontaktlinse angeordnet sind und durch das bewegte Lid je nach Lidposition abgedeckt sind. Eine einfache Ausführung zur Detektion von Lidschlüssen erfordert lediglich einen Sensor, der nur den Lidschluss, nicht aber die exakte Lidpösition erfasst. Ist die exakte Lidposition oder die Lidschlussgeschwindigkeit erwünscht, sind mindestens zwei Sensoren, die bei einem Lidschluss seriell überdeckt werden, erforderlich. Die Empfindlichkeit liegt im bevorzugten vom Auge sichtbaren Wellenlängenbereich vorzugsweise bei 400-500 nm. Bei lichtempflindlichen Sensoren (Fotosensoren) können jedoch Umwelteinflüsse (wie z.B. Flackern, Lichtblitze etc.) als' Blinzeln missinterpretiert werden. Als Gegenmaßnahme wird hier eine Begrenzung der max. Sensorabtastfrequenz z.B. auf ca. 10 Hz (vorzugsweise zwischen 5 bis 100 Hz) vorgeschlagen.

### 2. Alltägliche Verhaltensmuster

Alltägliche Verhaltensmuster umfassen die Fälle, bei denen das Auge und/oder das Lid entweder eine charakteristische Belastungs- oder Bewegungsabfolge erfährt. Allen gemein ist, dass sich der Akkommodationsbedarf über einen bestimmten Zeitraum sich nur unwesentlich ändert, d.h. keine Akkommodationsbedarfserfassung erforderlich ist. Vorgewählte Brennweitebereiche werden dabei vorzugsweise wiederholt oder über einen längeren bestimmten Zeitraum fest eingestellt oder zwischen zwei oder mehreren vorgegebenen Brennwerten hin- und her geschaltet. Diese Verhaltensmuster umfassen insbesondere Lesen, Bildschirmarbeit, Fernsehen, Autofahren, Ruhezustände oder Sport, wie z.B. Joggen.

Beim Lesen entstehen charakteristische, sprunghafte Bewegungsmuster mit sehr vielen Sakkadenbewegungen z.B. mit Lesen einer Zeile nach rechts und am Ende jeder Zeile eine große Sakkade nach links, ohne dass es einer großen Änderung in der Brechkraft des Akkommodationssystems bedarf. So lange dieses Sakkadenmuster detektiert wird, muss kein Akkommodationsbedarf angepasst werden. Das Steuersignal dient vorzugsweise als Stellsignal für einen vorgegebenen Brennweitenbereich (z.B. Tiefenschärfebereich um eine vorgegebene Brennweite), alternativ als Triggerung für eine einmalige oder mehrmalige Akkommodationsbedarfserfassung zur Einstellung dieses Brennweitenbereichs. Alternativ dient das Steuersignal als Startsignal für die Einstellung einer reduzierten Frequenz auf ca. 0,5 Hz für die Ackommodationserfassung im Akkommodationssystem (oder OTS). Als Sensor dienen vorzugsweise Beschleunigungssensoren auf dem Augapfel zur Erfassung der Augapfeldrehungen bei Blickrichtungswechseln.

Wie beim Lesen treten auch bei Suchaufgaben sehr charakteristische Augenbewegungen mit vielen Sakkaden auf, und zwar typischerweise in sehr kurzer Abfolge, aber im Unterschied zum Lesen ohne eine definierte Richtung. Lediglich wenn ein Punkt im Raum betrachtet wird und unscharf wirkt, finden kurzzeitig keine Sakkaden statt. Insofern stellen Suchaufgaben ein eigenes Verhaltensmuster dar, das bevorzugt von einem Beschleunigungssensor auf dem Augapfel detektierbar ist und das Steuersignal vorzugsweise eine Akkommodationsbedarfserfassung für die Einstellung eines danach einzuhaltenden Brennweitenbereichs auslöst. Eine Alternative sieht eine sensorische Ausstattung, Wertung und Reaktion auf Suchbewegungen entsprechend einer für Sakkaden vorgenannt vorgeschlagenen Ausführung vor.

Wie beim Lesen ist z.B. bei einer Bildschirmarbeit, beim Fernsehen, beim Autofahren, im Theater oder Kino oder auch beim Umherschweifen oder Beobachten über einen längeren Zeitraum nur eine Brennweiteneinstellung im Akkommodationssystem (oder OTS) erforderlich. Charakteristisch für diese Verhaltensmuster ist auch eine weitgehend unveränderte Blickrichtung insbesondere relativ zur Vertikalen. Für die Erfassung dieses Verhaltensmusters eignen sich vorzugsweise Beschleunigungssensoren alternativ oder ergänzt durch weitere Sensoren. Ändert sich die Position und Blickrichtung des Akkommodationssystemträgers relativ zur Umgebung nicht oder nur unwesentlich (z.B. bei einer Bildschirmarbeit, beim Fernsehen, im Theater, Kino oder beim Beobachten) umfassen diese weiteren Sensoren Magnetfeldsensoren oder Gyroskope, aber Licht- oder Farbsensoren für die jeweils zu beobachtenden Bilder oder Gegenstände. Ändert sich die Position und/oder Blickrichtung wie z.B. beim Autofahren laufend oder signifikant, reduzieren sich diese weiteren Sensoren vorzugsweise auf Licht- und Farbsensoren, die insbesondere größere Licht- und/oder Farbänderungen erfassen. Diese treten bei bestimmten Situationen auf, die oftmals eine besondere Aufmerksamkeit erfordern z.B. bei einer Einfahrt in einen Tunnel oder bei Lichtblendungen, Lichtschalterbetätigungen, Filmende oder andere von außen einwirkende Einflüsse. Diese bestimmten Situationen lassen sich im System eintrainieren oder einprägen bzw. in der zulässigen Referenzsignalbandbreite berücksichtigen (lernendes System). Sprechen diese weiteren Sensoren unabhängig vom Beschleunigungssensor an, erzeugen sie ein zusätzliches Messsignal und vorzugsweise auch ein Steuersignal, das z.B. eine oder mehrere Akkommodationsbedarfserfassungen einmalig auslöst und ggf. den vorgegebenen eingestellten Brennweitenbereich überprüft. Ferner eignen sich Licht- und Farbsensoren zur Erkennung von bestimmten Wellenlängenspektren oder optischen Impulsfolgen, die beispielsweise für bestimmte Bildschirme von Rechnern oder Fernsehgeräten während ihres Betriebs charakteristisch sind. Ansonsten ist eine Änderung der Brennweiteneinstellung in der Regel nur bei einem Verlassen oder Unterbrechen dieser Verhaltensmuster erforderlich, beispielsweise bei einem Blick auf die Uhr oder eines Tachometers.

Eine weitere Gruppe von Verhaltensmustern umfassen Tätigkeiten, bei denen das Auge als Ganzes bereits ohne eine Drehung in der Augenhöhle einem Beschleunigungsmuster innerhalb einer Referenzsignalbandbreite folgt. Zu dieser Gruppe zählt beispielsweise eine Vielzahl von Individual- und Mannschaftssportarten wie z. B. Joggen, Schwimmen, Radfahren, Ballspiele oder grundsätzlich auch alle anderen Tätigkeiten (wie Wandern, Bedienung von Motorfahrzeuge aller Art oder vibrierender Werkzeuge oder Geräte), bei denen der Kopf einer zyklischen oder anderweitig charakteristischen Beschleunigungsfolge ausgesetzt ist. Beispielsweise bei einem Gang wird dieses Beschleunigungsmuster durch die Schrittfrequenz (vorzugsweise zwischen 1 und 3 Hz) sowie durch die Beschleunigung in die Richtung des Schwerefeldes mit erhöhter Amplitude > 1 g umrissen. Als Sensor für die Erfassung dieser Beschleunigungsmuster eignen sich die vorgenannten Beschleunigungssensoren, deren Messsignale in der Signalerkennung der Erfassungseinheit mit den charakteristischen Referenzsignalbandbreiten verglichen werden und bei einer Überdeckung der Messsignale durch eine dieser Referenzsignalbandbreiten das Steuersignal auslöst. Vorzugsweise löst das Steuersignal als Triggersignal eine Aktivierung oder Deaktivierung einer Akkommodationsbedarfserfassung aus. Ist diese deaktiviert, erfolgt im Akkommodationssystem vorzugsweise ein Einfrieren des eingestellten Brennweite sowie des darum angeordneten Brennweitenbereichs (Schärfentiefebereich um die Brennweite), alternativ eine Einstellung dieser vorzugsweise im Fernbereich.

### 3. Bewusste Augen- und Lidschlagbewegungen

Bewusste Augenbewegungen umfassen die Fälle, bei denen das Auge und/oder das Lid eine charakteristische Bewegung oder Bewegungsfolge durchführen, die zu einem frei wählbaren Zeitpunkt bewusst, d.h. nicht reflexartig auf einen bewusst oder unbewusst hervorgerufenen Zustand angesteuert, herbeigeführt wird. Davon umfasst werden insbesondere bewusste Lidschläge oder Lidschlagfolgen und/oder ein bewusstes Drehen der Augäpfel.

Vorzugsweise sind dieses Bewegungen oder Bewegungsfolgen gegenüber den genannten unbewussten Augenbewegungen oder alltäglichen Verhaltensmustern oder auch gegenüber Umfeldsignalen hinreichend unterscheidungskräftig. Dies erfolgt vorzugsweise mittels eines unnatürlichen Bewegungsablaufs des Augapfels und / oder der Lider, ,z.B. über eine bestimmte Taktfolge des Lidschlags ähnlich eines einfachen Morsecodes einer oder beider Augen oder ein Anfahren bestimmter Augapfelausrichtungen.

Lidschläge oder Lidschlagfolgen sind für die Aktivierung oder Deaktivierung einer Akkommodationsbedarfserfassung nutzbar und dienen indirekt dem Einfrieren, der Einstellung oder der Änderung der Brechkraft des Akkommodationssystems, beispielsweise auch zur indirekten Umschaltung zwischen einer Nahsicht und einer Fernsicht. Im Rahmen einer Ausführungsform lassen sich durch das Steuersignal mit Deaktivierung der Akkommodationsbedarfserfassung zusätzlich auch Schaltzustände aktivieren, die Brennweitenverstellungen zulassen, z.B. eine Brennweitenänderung in voreingestellten Stufen, wobei jede Stufe separat durch ein Steuersignal ausgelöst wird. Auch sind durch Lidschläge oder Lidschlagfolgen auch bestimmte Betriebszustände wie eine automatische Akkommodationsmessung, ein Standby-Modüs, eine Erhöhung und/oder Absenkung der Mess-frequenz oder der Akkommodationsbedarfserfassung, aber auch ein einfaches Ein- und Ausschalten des Akkommodationssystems steuerbar.

Erfasst werden bewusste Lidschläge und Lidschlagfolgen durch die lichtempfindlichen Sensoren oder Schaltern, wie sie bereits im Rahmen der unbewussten Lidschläge zuvor beschrieben werden. Lidschläge und Lidschlagfolgen sind im Rahmen der Erfindung sowohl monokular und binokular erfassbar. Morsecodes oder eine andere Abfolge von bewussten Lidschlagbewegungen werden im Rahmen einer möglichen Ausführung beliebig auf beide Augen verteilt. Eine Auswertung binokularer Signale benötigt den Einsatz einer Kommunikationstrecke zwischen den Mitteln in beiden Augen. Vorzugsweise wird die Kommunikationsstrecke dabei pulsartig unmittelbar nach Auftreten eines Lidschlags oder einer Lidschlagfolge aktiviert.

Ein bewusstes Augenschließen, das wie zuvor beschrieben mit lichtempfindlichen Sensoren oder Schalter bevorzugt am Augapfel (alternativ im Augeninnern) erfassbar ist, eignet sich vorzugsweise zur Deaktivierung der Akkommodationsbedarfserfassung oder des gesamten Akkommodationssystems bei geschlossenen Augen. Die Deaktivierung erfolgt vorzugsweise nach Ablauf einer einstellbaren Zeitspanne nach Erfassung eines geschlossenen Lids, vorzugsweise nach typischerweise 2 bis 5 Sekunden.

Die Deaktivierung der Akkommodationsbedarfserfassung endet mit einer erneuten Aktivierung, vorzugsweise sofort mit einer erneuten Erfassung von Licht z.B. bei einer Öffnung der Augenlider oder bei Umgebungsdunkelheit mit dem ersten erfassbaren Licht. Geschlossene Augenlider werden bevorzugt durch die vorgenannten lichtempfindlichen Sensoren oder Schalter erfasst, wobei eine Auswertung des blauen Lichtspektrums (ca. 420-480 mm Wellenlänge, entspricht ca. 624 - 714 THz Frequenz) besonders vorteilhaft ist. Im blauen Lichtspektrum ist die Transmission des Augenlids am geringsten, während insbesondere rotes und Infrarotlicht ein Lid besonders gut durchdringt. Insofern eignet sich für die Erfassung eines geschlossenen Auges und der vorgenannten Augenbewegungen und damit für die vorgenannte Aktivierung und Deaktivierung der Akkommodationsbedarfserfassung vorzugsweise eine Erfassung von blauen Lichtbestandteilen.

Bewusste Augenbewegungen umfassen ferner bewusst herbeigeführte unnatürliche Augenbewegungen wie z.B. Schielen oder Augapfeldrehungen. (Augenpfadbewegungen) insbesondere bei geschlossenen Lidern. Diese lassen sich - wie zuvor angeführt - bevorzugt mit am Augapfel angebrachten Beschleunigungssensoren oder Magnetfeldsensoren erfassen. Werden ein Schielen (entspricht einem berechneten Akkommodationsbedarf über 3 dpt, vorzugsweise über 10 dpt) oder extreme Verdrehungen der Augen bevorzugt relativ zueinander (Vergenzwinkelmessung) oder relativ zum Kopf detektiert, lösen sie ein Triggersignal für eine Funktion im Akkommodationssystem aus, z.B. eine Herabsetzung der Frequenz zur Akkommodationsbedarfserfassung.

Bei Augenpfadbewegungen mit offenen oder geschlossenen Lidern oder auch in Kombination mit einer zeitlichen Lidschlagabfolge führt der Träger des Systems eine Augenbewegung entlang eines vorgegebenen Pfads oder Musters aus, die von den Sensoren erfassbar und von der Signalerkennung durch Vergleich mit einer Referenzsignalbandbreite erkennbar sind. Das daraus generierte Steuersignal dient der Triggerung für eine Funktion im Akkommodationssystem. Ein beispielhaft vorgegebener Augenpfad könnte mit einem Blick nach links'oben (l.o.) starten. Danach würde der Blick nach rechts unten (r.u.) schweifen, und dann links unten (l.u.) enden. Die Anzahl der Positionen im Pfad sind grundsätzlich variabel einstellbar und kann so hoch gewählt werden, dass keine Verwechslungsgefahr mit natürlichen Augenbewegungen mehr besteht.

### 4. Umfeldsignalfolge

Die Gruppe der Umfeldsignalfolgen umfassen die Fälle, bei denen die erfassbaren Signale nicht oder nicht allein durch Bewegungen der Augen und/oder der Lider generiert werden, sondern durch von außen aufgeprägte Signale erzeugt oder beeinflusst werden. Hierzu zählen insbesondere bestimmte charakteristische Lichtwellenlängen (z.B. bestimmte Lichtfarben z.B. eines Fernsehers oder eines Computerbild-schirms) oder Lichtintensitäten aus der Umgebung bis hin zu einer Umgebungsdunkelheit (z.B. bei Nacht). Die Lichtintensitäten werden entweder integral oder einzelne Wellenlängenbereiche selektiv vorzugsweise durch lichtempfindliche Sensoren oder Schalter (z.B. Fotosensoren, Fotodioden, CCD-Chips etc.) mit oder ohne Filter bevorzugt direkt auf der Augapfeloberfläche erfasst. Eine Ausführung sieht vor, die Sensoren oder Schalter in einer Kontaktlinse zu integrieren, wobei das Kontaktlinsenmaterial vom erfassten Licht durchschienen wird und dabei als optischer Filter dient.

Fällt bei einer einbrechenden Nacht oder bei sonstiger eintretender Dunkelheit die erfasste Lichtintensität bei offenen oder geschlossenen Augenlid unterhalb einer vorgebbaren Grenzintensität, wird dies durch die Signalerkennung der Erfassungseinheit erkannt und ein Steuersignal oder eine Steuersignalfolge erzeugt. Mit diesem Steuersignal wird vorzugsweise ein Ruhezustand eingestellt, d.h. die Akkommodationsbedarfserfassung unterbrochen oder auf eine reduzierte Wiederholungsfrequenz eingestellt. Eine Ausführung sieht zusätzlich zu der Unterbrechung eine Einstellung einer festen Brennweite vor. Übersteigt dann die erfasste Lichtintensität die Grenzintensität wieder, erfolgt über eine Generierung eines weiteren Steuersignals eine Beendigung des Ruhezustands.

Alternativ lassen sich mit einer Erfassung von charakteristischen Lichtfarben, wie sie z.B. beim Fernsehen, im Kino, bei der Computerarbeit oder im Straßenverkehr auftreten, Steuersignale für vorzugsweise eine voreingestellte Brennweite im Akkommodationssystem generieren. Beim Fernsehen beispielsweise ist der Betrachter durch die Schnitte im Film einem sehr charakteristischen Beleuchtungsverlauf ausgesetzt. Die Schnitte gehen meist mit einem Helligkeitssprung einher. Im Film tritt ein Schnitt durchschnittlich alle 1 bis 10 Sekunden auf. Innerhalb einer Sequenz, zwischen zwei Schnitten, stellt sich ein kontinuierlicher Helligkeitsverlauf durch das sich verändernde Bild ein. Die Farbtemperatur eines Fernsehbildes ist meist bläulich. Dies resultiert aus dem für die Bilddarstellung gewählten Farbraum sRGB mit einem Weißpunkt mit einer Farbtemperatur von 6500 K (Unterschied und Unterscheidungskriterium zu Tageslicht, ca. 5000 - 5800 K). Generell ist die Farbtemperatur für die Erkennung einer Umweltsignalfolge heranziehbar.

Die Erfindung erstreckt sich vorzugsweise auch auf ein Komplettsystem, d.h. ein Akkommodationssystem mit mindestens einem der vorgenannten Mittel zur Steuerung der Akkommodationsbedarfserfassung. Die Erfassungseinheit ist hierfür vorzugsweise über die Signalerkennung mit dem Informationsverarbeitungssystem des künstlichen Akkommodationssystems verbunden und überträgt'über diese Verbindung das Steuersignal zur Beeinflussung der Akkommodationsbedarfserfassung und damit des Regelkreises zur Akkommodationssteuerung.

Das Akkommodationssystem ist vorzugsweise ein akkommodierendes Intraokularlinsensystem (IOL), d.h. ein Implantat zur Substituierung oder Ergänzung der künstlichen Linse oder ein Kontaktlinsensystem, das als Akkommodationssystem auf ein Auge aufgesetzt wird. Insbesondere wenn das Akkommodationssystem ein Implantat ist und die Mittel zur Steuerung der Akkommodationsbedarfserfassung z.B. über ein Kontaktelement oder Kontaktlinse außerhalb des Augapfels z.B. auf das Auge aufgesetzt ist, besteht die vorgenannte Verbindung zur Übertragung des Steuersignals vorzugsweise auf der Basis von optischen oder elektromagnetischen Übertragungswegen oder einer Funkverbindung.

Die Erfindung erstreckt sich ferner auf eine Verwendung der vorgenannten Mittel zur Steuerung der Akkommodationsbedarfserfassung für ein Akkommodationssystem sowie auf ein Verfahren zur Steuerung der Akkommodationsbedarfserfassung. Dabei werden insbesondere eine körpereigene Signalfolge oder eine Umfeldsignalfolge mit mindestens einem Sensor erfasst und durch diesen in Messsignale gewandelt. Die Messsignale werden dann vorzugsweise in einer Signalerkennung in einer Erfassungseinheit mit einer Referenzsignalbandbreite verglichen und bei einer Überdeckung der Messsignale durch eine vorgegebene Referenzsignalbandbreite das Steuersignal erzeugt.

Das System ist monokular oder binokular gestaltbar. Bei einem binokularen System wird je ein System auf jedes Auge positioniert, wobei die Systeme untereinander vorzugsweise drahtlos kommunizieren und insbesondere Messwerte und Signale zur Steuerung der Akkommodationsbedarfserfassung austauschen oder zusammenfassen auch Aufgaben aufteilen.

Die Erfindung wird im Folgenden anhand von Ausführungsbeispielen und Details mit Figuren näher erläutert, die optional auch mit einzelnen oder allen vorgenannten Maßnahmen zusätzlich kombinierbar oder erweiterbar sind. Es zeigen
**Fig.1a** **und b** Blockdiagramme der Komponenten der beanspruchten Mittel zur Steuerung der Akkommodationsbedarfserfassung beispielhaft für ein Auge,
**Fig.2** eine Schaltung zur Detektion einer körpereigenen Signalfolge,
**Fig.3** eine erste binokulare Ausführung für beide Augen mit zwei Erfassungssystemen und bidirektionalem Datenaustausch sowie
**Fig. 4a** **und b** weitere binokulare Ausführungen für beide Augen und einem gemeinsamen Erfassungssystem.

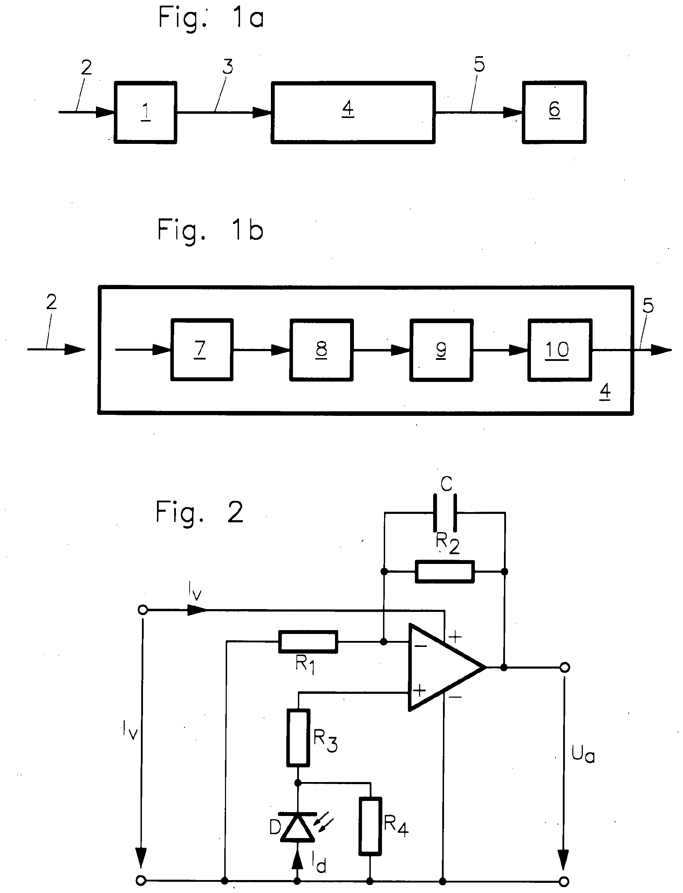

Die Mittel zur Steuerung der Akkommodationsbedarfserfassung umfassen in einer in **Fig.1a** dargestellten Ausführungsform einen Sensor **1** (Signalaufnahme), zur Erfassung einer Signalfolge **2** und Wandlung dieser, in Messsignale **3.** Diese werden einer Erfassungseinheit **4** zugeführt und in dieser zu einem Steuersignal **5** zur Beeinflussung eines Akkommodationssystems **6** oder eines OTS verarbeitet. Vorzugsweise sind alle genannten Komponenten in das Akkommodationssystem oder OTS integriert, entweder als Implantat oder integriert in eine intelligente Kontaktlinse und vorzugsweise ohne zusätzliche externe Komponenten.

**Fig.1b** gibt das Blockschaltbild der Erfassungseinheit **4** im Detail wieder. Die Messsignale **3** werden zunächst vorzugsweise einer Segmentierung **7** zugeführt. Die Segmentierung stellt einen optionalen ersten Signalverarbeitungsschritt dar, durch welchen die Augen- und Lidschlagbewegungen in einzelne charakteristische bevorzugt zeitliche Segmente unterteilt werden. Die Segmentierung vereinfacht die nachfolgende Signalverarbeitung, indem diese für eine Signalerkennung sich optional auf bestimmte Segmente beschränken kann. Der Aufwand und damit der Energiebedarf der Signalverarbeitung werden damit vorteilhaft reduziert. Alternativ ermöglichen trainingsdatenbasierte Entscheidungsnetzwerke (z.B. künstliche neuronale Netze) eine integrale Signalverarbeitung, ohne dass es einem Segmentierungsschritt als eigenen vorangeschalteten Schritt bedarf.

Die Messsignale werden segmentiert oder unsegmentiert (direkt) einer Signalerkennung **8** zugeführt. Die Signalerkennung ermöglicht eine Interpretation der Segmente bezüglich vordefinierter Bewegungsmuster, indem sie die Messsignale mit Referenzsignalbandbreite vergleicht und eine Überdeckung der Messsignale durch eine vorgegebene Referenzsignalbandbreite erkennt. Insbesondere erfolgt in diesem Signalverarbeitungsschritt eine Differenzierung zwischen natürlichen Bewegungen und solchen Bewegungen, die der Nutzer bewusst zum Zwecke der Steuerung ausübt. Wird ein Bewegungsmuster erkannt, wird dies als Signal zu einem Auslöser **9** weitergeleitet. Der Auslöser dient der Zuordnung eines Bewegungsmusters zu einer definierten Steueraktion. Er erfüllt somit die Aufgabe einer Ereignistriggerung für eine Steueraktion **10**. Die Steueraktion erfüllt die Umsetzung des Steuerbefehls, d.h. die Erzeugung des Steuersignals **5** z.B. für die Steuerung der Akkommodationsbedarfserfassung.

**Fig.2** zeigt im Rahmen der Signalaufnahme beispielhaft eine Verstärkerschaltung zur Erfassung einer körpereigenen Signalfolge wie dem Lidschluss. Sie zeichnet sich durch die Verwendung eines Ultra-Low-Power Operationsverstärkers als Tiefpassfilter und gleichzeitig als Vorverstärker für den verwendeten Lichtsensor (Photodiode) aus und ermöglicht mit diesen Merkmalen nicht nur besonders geringe Baugrößen, sondern auch einen geringen Energiebedarf.

Des Weiteren sind die Mittel sowie ein Verfahren zur Steuerung der Akkommodationsbedarfserfassung in einem künstlichen Akkommodationssystem oder einem OTS neben einer monokularen Anwendung (Erfassung der Signale in einem Auge) auch binokular (Erfassung der Signale in beiden Augen) anwendbar. Somit können z.B. Blinzelmuster erkannt werden, die einen kombinierten Lidschlag und/oder Rotationsbewegungen beider Augen umfassen.

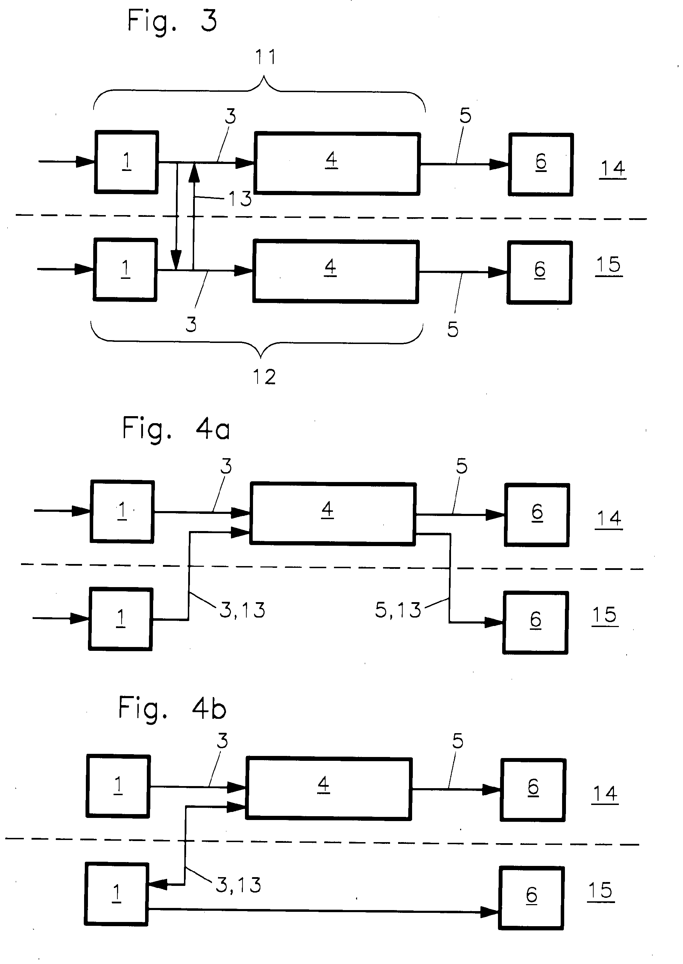

Eine erste Ausführungsform für eine binokulare Anwendung offenbart **Fig.3****.** Bei dieser existiert neben den ersten Mitteln **11** im oder am ersten Auge **14** ein zweites OTS mit zweiten Mitteln **12** zur Steuerung der Akkommodationsbedarfserfassung im oder am zweiten Auge **15,** umfassend mindestens einen Sensor 1 zur Erfassung einer körpereigenen Signalfolge. Damit erfolgt eine Signalerfassung in beiden Augen. Vorzugsweise sind das künstlichen Akkommodationssystem oder das OTS sowie weiter bevorzugt die Mittel zur Steuerung der Akkommodationsbedarfserfassung in beiden Augen identisch. Ebenso bevorzugt erfolgt die Akkommodationsbedarfserfassung redundant. Zum Zwecke der Signalerkennung findet ein uni- bzw. bidirektionaler Datenaustausch **13** zwischen den beiden Mitteln **11** und **12** statt, vorzugsweise der Messsignale **3** zwischen Sensor **1** und Erfassungseinheit **4**.

Weitere Ausführungsformen für eine binokulare Anwendung offenbaren **Fig.4a** **und b.** Diese zeichnen sich dadurch aus, dass das erste und zweite Auge jeweils einen eigenen Sensor **1**, und ein eigenes Akkommodationssystem **6** (oder OTS) aufweist, die jedoch mit einer gemeinsamen Erfassungseinheit **4** kommunizieren. Die Systeme sind vorzugsweise nicht redundant. Sie teilen sich vielmehr die Aufgaben auf. Vorzugsweise ist die Erfassungseinheit in einem der beiden Augen (im Beispiel erstes Auge **14)** positioniert und kommuniziert vorzugsweise drahtlos mit dem Sensor und dem Akkomodationssystem des anderen Auges (im Beispiel zweites Auge **15).** Während **Fig.4a** eine Ausführung zeigt, bei der die gemeinsame Erfassungseinheit **4** direkt mit dem Sensor und dem Akkommodationssystem im jeweils anderen Auge kommuniziert, nutzt die in **Fig.4b** dargestellte Ausführung den Sensor des jeweils anderen Auges als alleiniges Übertragungsmittel für einen Datenaustausch zur zentralen Erfassungseinheit. Das Akkommodationssystem **6** des zweiten Auges **15** erhält dann das Steuersignal mittelbar über den Sensor.

Binokulare Anwendungskonzepte umfassen nicht folglich nur einen parallelen Einsatz von zwei identischen Mitteln zur Steuerung der Akkommodationsbedarfserfassung in einem, ophthalmischen technischen System mit oder ohne dem vorgenannten Datenaustausch, sondern auch einen Einsatz von zwei unterschiedlichen Mitteln oder einem Mittel insbesondere mit unterschiedlichen Sensoren zur getrennten Erfassung z.B. von Lidreflex und Augenausrichtung an unterschiedlichen Augen. Dabei dienen vorzugsweise die Sensoren als Sender und Empfänger für einen drahtlosen uni- oder bidirektionalen Datenaustausch.

### Bezugszeichenliste:

- 1: Sensor
- 2: Signalfolge
- 3: Messsignal
- 4: Erfassungseinheit
- 5: Steuersignal
- 6: Akkommodationssystem
- 7: Segmentierung
- 8: Signalerkennung
- 9: Auslöser
- 10: Steueraktion
- 11: erstes Mittel
- 12: zweites Mittel
- 13: Datenaustausch
- 14: erstes Auge
- 15: zweites Auge

## Patentansprüche

1. Verwendung von Mitteln für eine Erzeugung von Steuersignalen zur Einstellung oder Anregung von Betriebszuständen in einem ophthalmischen technischen System **(6),** umfassend
a) mindestens einen Sensor **(1)** für eine Erfassung einer körpereigenen Signalfolge oder einer Umfeldsignalfolge und Wandlung dieser in Messsignale **(3),**
b) mindestens eine Erfassungseinheit **(4)** für eine Wandlung der Messsignale **(3)** in ein Steuersignal **(5)**, das die Akkommodationsbedarfserfassung beeinflusst,
wobei
c) die Erfassungseinheit **(4)** eine Signalerkennung **(8)** aufweist, die die Messsignale **(3)** mit Referenzsignalen vergleicht, die innerhalb einer Referenzsignalbandbreite liegen, und das Steuersignal **(5)** erzeugt
**dadurch gekennzeichnet, dass**
d) mindestens einer der Sensoren **(1)** ein Beschleunigungssensor, ein Magnetfeldsensor und/oder ein Gyroskop ist,
e) die Einstellung oder Anregung von Betriebszuständen eine Einstellung von Eigenschaften einer Akkommodationsbedarfserfassung beinhaltet sowie
f) die körpereigene Signalfolge aus einer Wiedergabe unbewusster Sakkadenbewegungen und/oder bewusster Augenbewegungen besteht und das Steuersignal **(5)** ein Triggersignal für die oder eine Einstellung der Eigenschaften der Akkommodationsbedarfserfassung ist.

2. Verwendung nach Anspruch 1, **dadurch gekennzeichnet, dass** die körpereigene Signalfolge eine Wiedergabe alltäglicher Verhaltensmuster oder die Umfeldsignalfolge eine Wiedergabe von Umfeldeinflüssen umfasst und das Steuersignal **(5)** ein Stellsignal für einen vorgegebenen Brennweitenbereich oder eine vorgegebene Frequenz in der Akkommodationsbedarfserfassung im ophthalmischen technischen System ist.

3. Verwendung nach einem der vorgenannten Ansprüche, **dadurch gekennzeichnet, dass** die Messsignale **(3)** Beschleunigungssignale oder Orientierungssignale sind, die mittels der Signalerkennung mit den charakteristischen Referenzsignalbandbreiten verglichen werden und bei einer Überdeckung der Messsignale durch eine dieser Referenzsignalbandbreiten das Steuersignal auslöst.

4. Verwendung nach Anspruch 3, **dadurch gekennzeichnet, dass** das Steuersignal **(5)** ein Triggersignal oder eine Triggersignalfolge für die Akkommodationsbedarfserfassung oder für die Einstellung einer festen Brennweite im ophthalmischen technischen System umfasst.

5. Verwendung nach Anspruch 4, **dadurch gekennzeichnet, dass** die Triggersignalfolge mindestens drei Triggersignale mit zunehmenden zeitlichen Abstand zueinander umfasst.

6. Verwendung nach einem der vorgenannten Ansprüche, **dadurch gekennzeichnet, dass** mindestens einer der Sensoren **(1)** ein Detektor zur Erfassung der Umfeldleuchtdichte und/oder des Lidschlags ist.

7. Verwendung nach einem der vorgenannten Ansprüche, umfassend zwei mittels Datenaustausch **(13)** miteinander kommunizierende Sensoren.

8. Verwendung nach einem der vorgenannten Ansprüche, **dadurch gekennzeichnet, dass** die Mittel mit einem ophthalmischen technischen System auf einer Kontaktlinse zum Einsatz kommen.

## Claims

1. Use of means for generating control signals for adjusting or initiating operational states in an ophthalmic technical system (6), comprising:
a) at least one sensor (1) for detection of an endogenous signal sequence or an environmental signal sequence, and converting of these into measurement signals (3),
b) at least one detection unit (4) for converting the measurement signals (3) into a control signal (5), which influences the accommodation demand detection,
wherein
c) the detection unit (4) comprises a signal recognition element (8), which compares the measurement signals (3) with reference signals, which lie within a reference signal bandwidth, and generates the control signal (5),
**characterised in that**
d) at least one of the sensors (1) is an acceleration sensor, a magnetic field sensor, and/or a gyroscope,
e) the adjustment or initiation of operational states includes an adjustment of properties or characteristics of an accommodation demand detection, and
f) the endogenous signal sequence consists of a reproduction of unknown saccade movements and/or known eye movements, and the control signal (5) is a trigger signal for the adjustment or an adjustment of the properties or characteristics of the accommodation demand detection.

2. Use according to claim 1, **characterised in that** the endogenous signal sequence comprises a reproduction of everyday behaviour patterns, or the environmental signal sequence comprises a reproduction of environmental influences, and the control signal (5) is an actuating signal for a predetermined focal length range or a predetermined frequency in the accommodation demand detection in the ophthalmic technical system.

3. Use according to any one of the preceding claims, **characterised in that** the measurement signals (3) are acceleration signals or orientation signals, which by means of the signal recognition unit are compared with the characteristic reference signal bandwidths, and, in the event of coverage of the measurement signals by one of these reference signal bandwidths, triggers the control signal.

4. Use according to claim 3, **characterised in that** the control signal (5) comprises a trigger signal or a trigger signal sequence for the accommodation demand detection or for the adjustment or setting of a fixed focal length in the ophthalmic technical system.

5. Use according to claim 4, **characterised in that** the trigger signal sequence comprises at least three trigger signals with increasing time intervals between one another.

6. Use according to any one of the preceding claims, **characterised in that** at least one of the sensors (1) is a detector for detecting the environmental light density and/or the eyelid blink.

7. Use according to any one of the preceding claims, comprising two sensors which communicate with one another by means of data exchange (13).

8. Use according to any one of the preceding claims, **characterised in that** the means are used with an ophthalmic technical system on a contact lens.

## Revendications

1. Utilisation de moyens d'obtention de signaux de commande pour permettre de régler ou de stimuler des états de fonctionnement dans un système technique ophtalmologique (6) comprenant :
a) au moins un capteur (1) permettant de détecter une succession de signaux propres à un corps ou une succession de signaux d'environnement et de les convertir en signaux de mesure (3),
b) au moins une unité de détection (4) permettant de convertir les signaux de mesure (3) en un signal de commande (5) qui agit sur la détection d'un besoin d'accommodation,
c) l'unité de détection (4) comprenant une reconnaissance de signaux (8) qui compare les signaux de mesure (3) avec des signaux de référence qui sont situés dans une largeur de bande de signaux de référence et produit le signal de commande (5),
**caractérisée en ce que**
d) au moins l'un des capteurs (1) est un capteur d'accélération, un capteur de champ magnétique et/ou un gyroscope,
e) le réglage ou la stimulation d'états de fonctionnement comprend un réglage de propriétés de la détection d'un besoin d'accommodation, et
f) la succession de signaux propres à un corps est constituée par la reproduction de mouvements saccadés involontaires et/ou de mouvements des yeux volontaires et le signal de commande (5) est un signal de déclenchement du ou d'un réglage des propriétés de la détection d'un besoin d'accommodation.

2. Utilisation conforme à la revendication 1,
**caractérisée en ce que**
la succession de signaux propres à un corps comporte la reproduction de modèles de comportements quotidiens, ou la succession de signaux d'environnement comporte la reproduction d'influences de l'environnement et le signal de commande (5) est un signal de réglage d'une plage de distances focales prédéfinie, ou une fréquence prédéfinie de la détection d'un besoin d'accommodation dans le système technique ophtalmologique.

3. Utilisation conforme à l'une des revendications précédentes,
**caractérisée en ce que**
les signaux de mesure (3) sont des signaux d'accélération ou des signaux d'orientation qui sont comparés au moyen de la reconnaissance de signaux avec les largeurs de bande caractéristiques du signal de référence, et en cas de chevauchement des signaux de mesure par l'une de ces largeurs de bande du signal de référence, le signal de commande est déclenché.

4. Utilisation conforme à la revendication 3,
**caractérisée en ce que**
le signal de commande (5) est un signal de déclenchement ou une succession de signaux de déclenchement de la détection d'un besoin d'accumulation ou du réglage d'une distance focale fixe dans le système technique ophtalmologique.

5. Utilisation conforme à la revendication 4,
**caractérisée en ce que**
la succession de signaux de déclenchement comporte au moins trois signaux de déclenchement ayant une distance croissante dans le temps.

6. Utilisation conforme à l'une des revendications précédentes,
**caractérisée en ce qu'**
au moins l'un des capteurs (1) est un détecteur permettant de détecter la luminance de l'environnement et/ou les clignements des yeux.

7. Utilisation conforme à l'une des revendications précédentes,
comportant deux capteurs communiquant l'un avec l'autre par échange de données (13).

8. Utilisation conforme à l'une des revendications précédentes,
**caractérisée en ce que**
les moyens sont mis en œuvre avec un système technique ophtalmologique sur une lentille de contact.
